# DEMANDE DE BREVET EUROPEEN

(11) **EP 2 438 932 A1**
(43) Date de publication de la demande: **11.04.2012**
(21) Numéro de dépôt: 10186604.4
(22) Date de dépôt: 05.10.2010
(51) Int. Cl.: A61L 2/18

(54) **Dispositif de désinfection pour les mains et les pieds**

(71) Demandeur: Adolph, Barbara, 1213 Petit-Lancy (CH)
(72) Inventeur: Adolph, Barbara, 1213 Petit-Lancy (CH)
(74) Mandataire: Bugnion Genève

(57) **Abrégé**

Dispositif de désinfection pour les pieds et les mains comprenant un compartiment supérieur dans lequel un utilisateur peut introduire les mains, un compartiment inférieur dans lequel il peut introduire les pieds et des buses de pulvérisation logées au sein des deux compartiments diffusant un produit désinfectant.

## Description

Le domaine de l'invention est celui des dispositifs de désinfection. L'invention porte en particulier sur un dispositif de désinfection pour les mains et les pieds.

De nos jours, suite à l'évolution des mouvements de population, suite à la mondialisation et à l'évolution grandissante des moyens de communication, la transmission de maladies dues à des bactéries ou des microbes présents dans l'air ambiant est grandement facilitée. Des maladies apparues dans un coin du globe peuvent se transmettre sur de longues distances via les différents canaux de communication (cargos, avions, etc.) pour dans certains cas être à l'origine de pandémies. Il s'avère donc de plus en plus nécessaire de proposer des dispositifs de désinfection adéquats face à ce type menace grandissante.

Le brevet japonais JP2009022561 décrit un dispositif de désinfection pour les semelles de chaussures. Ce dispositif ne présente cependant aucun moyen permettant la désinfection des mains.

Le brevet américain US 5,714,119 présente un dispositif de désinfection pour les semelles de chaussure et les mains. Ce dispositif présente toutefois un désavantage majeur en termes de sécurité d'utilisation. Rien n'empêche en effet un enfant d'introduire les mains ou les pieds dans le dispositif qui se met alors en fonction immédiatement. Sans que cela soit voulu, l'enfant peut alors être aspergé de produit désinfectant ce qui peut être dangereux si le produit entre en contact avec les muqueuses ou les yeux de l'enfant.

L'un des buts de l'invention est donc de fournir un dispositif de désinfection pour les mains et les pieds. Un autre but de l'invention est de fournir un dispositif de désinfection dont la mise en fonction par un enfant est limitée.

Dans ces buts, l'invention propose un dispositif de désinfection pour les pieds et les mains tel que défini par la revendication 1.

Des modes de réalisation avantageux sont illustrés dans les revendications dépendantes.

L'invention sera mieux comprise par l'homme du métier grâce à la description détaillée suivante en relation avec les dessins accompagnants.
La figure 1 montre une vue en perspective de face du dispositif de désinfection selon l'invention.
La figure 2 est une vue schématique du circuit électrique du dispositif de désinfection selon l'invention.
La figure 3 montre le circuit hydraulique du dispositif de désinfection selon l'invention.

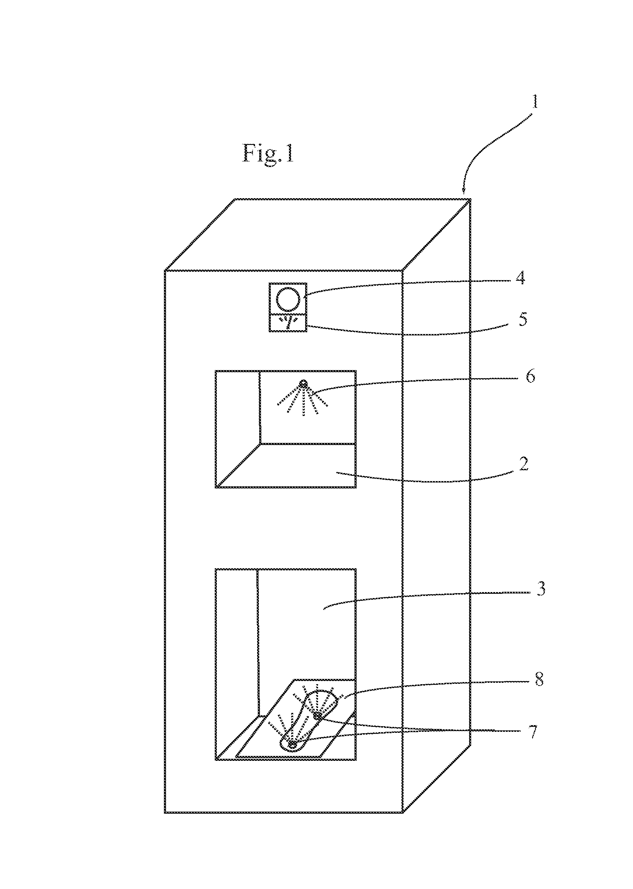

Le dispositif de désinfection pour les pieds et les mains montré par la figure 1 comprend un bâti 1 de forme parallélépipédique. Le bâti peut toutefois avoir toute forme oblongue. Le bâti comprend un compartiment supérieur 2 dans lequel l'utilisateur peut introduire les mains et un compartiment inférieur 3 dans lequel l'utilisateur peut introduire un pied. Au sens de la présente invention, on entend par le terme « pied », aussi bien le pied humain nu que le pied chaussé d'une chaussure. Le bâti du dispositif est de préférence d'une hauteur supérieure à 1.4 m.

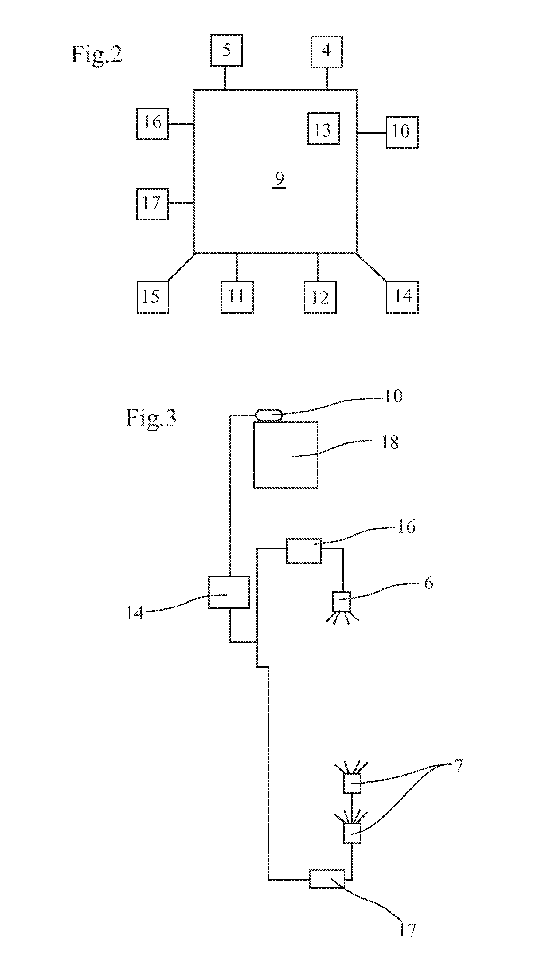

Le dispositif comprend au dessus du compartiment supérieur un moyen d'activation 4. Le moyen d'activation 4 peut consister en un bouton poussoir ou un capteur de proximité. Dans le cas d'un capteur, ce dernier peut consister en un capteur infrarouge, capacitif, inductif, à effet Hall ou tout autre type de capteur bien connu de l'homme du métier. Le moyen d'activation 4 est représenté sur la figure 1 comme étant situé sur la face avant du bâti mais il peut également être logé sur une autre face du bâti. Le moyen d'activation est situé à une hauteur suffisante pour qu'un enfant ne puisse facilement l'atteindre. Le moyen d'activation est préférablement situé à une hauteur supérieure à 1.3 m. Lorsque l'utilisateur désire utiliser le dispositif de désinfection, il doit tout d'abord activer ce dernier en utilisant le moyen d'activation 4. Suite à cette opération effectuée par l'utilisateur, le moyen d'activation transmet un signal d'activation à l'unité de contrôle 9 du dispositif (représentée figure 2).

Le dispositif comprend un moyen d'affichage 5 monté sur le bâti 1 et fournissant une indication de l'état de remplissage en produit désinfectant du dispositif. Comme représenté sur la figure 1, cet affichage peut consister en un affichage analogique présentant une échelle graduée sur laquelle se déplace une aiguille indiquant la quantité de produit désinfectant présente à l'intérieur du dispositif. Cet affichage peut alternativement consister en un témoin lumineux, par exemple clignotant plus ou moins vite en fonction de l'état de remplissage du dispositif. Le moyen d'affichage peut également consister en un affichage numérique.

Le compartiment supérieur 2 du dispositif comprend une buse de pulvérisation 6 permettant de diffuser un produit désinfectant stocké dans le dispositif à l'intérieur du compartiment supérieur afin de désinfecter les mains d'un utilisateur. Le compartiment supérieur peut toutefois comprendre plus d'une buse de pulvérisation. Le compartiment supérieur comprend également des premiers moyens de détection, par exemple un ou plusieurs capteurs de présence 11 (représenté fig. 2), permettant de détecter la présence des mains de l'utilisateur au sein du compartiment. Le ou les capteurs de présence peuvent être des capteurs infrarouges, capacitifs, inductifs, à effet Hall ou tout autre type de capteur bien connu de l'homme du métier. Lorsque l'utilisateur introduit les mains dans le compartiment supérieur, le ou les capteurs détectent la présence des mains de l'utilisateur et transmettent un premier signal de présence à l'unité de contrôle 9.

Le compartiment inférieur 3 du dispositif comprend deux buses de pulvérisations 7 situées en dessous d'une pédale mobile 8. Tel que représenté sur les figures 1 et 3, le compartiment inférieur comprend donc deux buses de pulvérisation. Toutefois, le compartiment inférieur peut alternativement comprendre trois ou quatre buses de pulvérisation. La pédale mobile 8 présente des ouvertures au niveau des buses de pulvérisation 7 afin de permettre la diffusion de produit désinfectant sous les pieds de l'utilisateur et au sein du compartiment inférieur. La pédale 8 se déplace entre une position de repos et une position de travail sous l'effet d'une pression du pied exercée par utilisateur. La pédale 8 comprend des moyens (non représentés) permettant le rappel de la pédale dans la position de repos lorsqu'aucune pression n'est exercée. Ces moyens peuvent consister en un ou plusieurs ressorts, des pièces construites dans un matériau à mémoire de forme, etc. Lorsque l'utilisateur impose une pression sur la pédale, celle-ci se déplace dans la position de travail et active des deuxièmes moyens de détection tels qu'un interrupteur 12 ou un relais électromécanique monté dans le bâti. Lorsque l'interrupteur ou le relais est activé, ce dernier transmet un deuxième signal de présence à l'unité de contrôle 9 du dispositif. Lorsque l'utilisateur relâche la pression sur la pédale, celle-ci regagne sa position de repos ce qui a pour conséquence de désactiver l'interrupteur 12 (ou le relais). Le deuxième signal de présence n'est alors plus transmis à l'unité de contrôle 9. L'utilisation d'une pédale contrairement à un capteur de présence permet d'éviter qu'un animal ne puisse, du fait de sa simple présence dans le compartiment inférieur, activer la pulvérisation de produit désinfectant.

La figure 2 montre un schéma du circuit électrique présent à l'intérieur du dispositif. Le dispositif comprend une unité de contrôle 9 permettant de commander une pompe 14 en fonction des signaux reçus du moyen d'activation 4, des premiers moyens de détection 11, des deuxièmes moyens de détection 12 et de moyens de temporisation 13.

L'unité de contrôle est raccordée au moyen d'affichage 5 indiquant l'état de remplissage du dispositif. L'unité de contrôle 9 est raccordée au moyen d'activation 4 dont elle reçoit le signal d'activation lorsqu'un utilisateur active le moyen d'activation (par pression si le moyen d'activation est un bouton poussoir ou en passant la main à proximité dans le cas ou le moyen d'activation consiste en un capteur de proximité).

L'unité de contrôle 9 est raccordée à des moyens de mesure 10 de l'état de remplissage du dispositif tels qu'une ou plusieurs jauges logées dans le réservoir 18 de produit désinfectant (représenté figure 3). Grâce à ces moyens l'unité de contrôle est renseignée en permanence de l'état de remplissage du dispositif. L'unité de contrôle met à jour le moyen d'affichage 5 en fonction des informations reçues des moyens de mesures 10.

L'unité de contrôle est raccordée au capteur de présence 11 logé dans le compartiment supérieur dont elle reçoit le premier signal de présence. L'unité de contrôle est également raccordée à l'interrupteur 12 qui, lorsque l'utilisateur imprime une pression sur la pédale pour la déplacer en position de travail, transmet le deuxième signal de présence à l'unité de contrôle. L'unité de contrôle est de plus raccordée à la pompe 14 qu'elle active en fonction des différents signaux reçus.

L'unité de contrôle 9 comprend de plus des moyens de temporisation 13 permettant de désactiver le dispositif après un temps donné. Les moyens de temporisation 13 permettent par exemple de désactiver le dispositif si après réception du signal d'activation l'unité de contrôle 9 ne reçoit aucun signal de présence. A cette fin une horloge, interne à l'unité de contrôle tel que représenté sur la figure 2 mais pouvant également être indépendante de celle-ci, permet de mesurer le temps écoulé depuis la réception du signal d'activation. Ainsi, si un utilisateur active le dispositif mais s'en va avant de l'avoir utilisé pour se désinfecter les pieds ou les mains, le dispositif se désactive automatiquement de lui-même après un certain temps enregistré dans l'unité de contrôle par l'installateur. Ceci limite par exemple le risque qu'un enfant utilise le dispositif alors que celui-ci a été activé par un autre utilisateur ayant quitté les lieux. Les moyens de temporisation 13 peuvent également désactiver le dispositif automatiquement après utilisation de celui-ci lorsqu'un temps prédéterminé, enregistré dans l'unité de contrôle par l'installateur, s'est écoulé après pulvérisation de produit désinfectant dans l'un des compartiments.

L'unité de contrôle est de plus raccordée à deux doseurs 16 et 17, eux-mêmes raccordés aux buses de pulvérisation 6 et 7, qu'elle pilote afin de diffuser le produit désinfectant par les buses de pulvérisation. Les paramètres de pulvérisation tels que le débit, le temps et la pression de pulvérisation sont enregistrés dans l'unité de contrôle qui agit sur les doseurs 16 et 17 pour diffuser le produit désinfectant selon ces paramètres de pulvérisation. Ces paramètres peuvent toutefois être modifiés à volonté par l'installateur en agissant sur l'unité de contrôle 9.

L'unité de contrôle 9 est raccordée à un moyen d'alimentation 15 permettant d'alimenter en énergie le dispositif. Le moyen d'alimentation peut consister en une prise permettant de raccorder le dispositif au secteur mais peut également consister en une batterie, une pile sèche ou un panneau photovoltaïque. L'unité de contrôle alimente la pompe 14, les doseurs 16 et 17, les capteurs de présence 11, le relais électromécanique 12 et le moyen d'affichage 5.

La figure 3 représente le circuit hydraulique permettant la diffusion de produit désinfectant. Le dispositif comprend un ou plusieurs réservoirs 18 munis des dispositifs de mesure 10 et permettant de stocker le produit désinfectant. Dans un mode d'exécution préféré, le dispositif contient un seul réservoir et une seule pompe mais il peut comprendre deux réservoirs et pompes ou plus, en particulier pour diffuser des produits différents dans les compartiments supérieurs et inférieurs. La pompe 14 est raccordée au réservoir 18 et aux doseurs 16 et 17 pilotés par l'unité de contrôle. Chacun des doseurs est raccordé à une ou plusieurs buses de pulvérisation.

Ainsi en fonctionnement, lorsque l'unité de contrôle 9 reçoit le signal d'activation et l'un des signaux de présence, le premier et/ou le deuxième, la pompe est activée et le produit désinfectant est pulvérisé dans le/les compartiment(s) correspondant(s). En revanche, si l'unité de contrôle ne reçoit pas le signal d'activation, le dispositif reste désactivé et ce même si un utilisateur introduit les mains ou le pied à l'intérieur d'un des compartiments. Il est donc nécessaire dans tous les cas, d'activer d'abord le dispositif grâce au moyen d'activation pour pouvoir ensuite utiliser le dispositif. Ceci présente une première sécurité limitant le risque qu'un enfant mette le dispositif en fonction simplement en introduisant les mains dans le compartiment supérieur ou un pied dans le compartiment inférieur.

De plus, vu que le moyen d'activation est situé en hauteur sur le bâti, de préférence à une hauteur supérieure à 1.3 m, la mise en fonction du dispositif par un enfant en est d'autant plus limitée. Ceci représente une sécurité supplémentaire pour éviter qu'un enfant échappant à la surveillance des adultes puisse activer le dispositif de désinfection et déclencher la pulvérisation.

## Revendications

1. Dispositif de désinfection pour les pieds et les mains comprenant un bâti, le bâti comprenant, dans sa partie supérieure, un compartiment supérieur (2) permettant à un utilisateur d'introduire les mains et, dans sa partie inférieure, un compartiment inférieur (3) permettant à un utilisateur d'introduire au moins un pied, un moyen d'alimentation (15) apte à alimenter en énergie le dispositif, une pompe (14), un moyen de stockage d'un produit désinfectant (18), une unité de contrôle (9), un moyen d'activation (4) apte à transmettre un signal d'activation à l'unité de contrôle afin d'activer le dispositif, des premiers moyens de détection (11) aptes à détecter la présence des mains d'un utilisateur dans le compartiment supérieur et à transmettre un premier signal de présence à l'unité de contrôle, des seconds moyens de détection (8, 12) aptes à détecter la présence du pied d'un utilisateur dans le compartiment inférieur et à transmettre un deuxième signal de présence à l'unité de contrôle, des premiers moyens de pulvérisation (6, 16) aptes à diffuser le produit désinfectant dans le compartiment supérieur, des seconds moyens de pulvérisation (7, 17) aptes à diffuser le produit désinfectant dans le compartiment inférieur, l'unité de contrôle étant apte à recevoir le signal d'activation, le premier signal de présence et le second signal de présence,
**caractérisé en ce que** l'unité de contrôle enclenche la pompe uniquement suite à la réception du signal d'activation et du premier et/ou deuxième signal de présence.

2. Dispositif selon la revendication 1, **caractérisé en ce que** le moyen d'activation (4) est situé à une hauteur choisie de sorte qu'un enfant puisse difficilement atteindre ledit moyen, en particulier à une hauteur supérieure à 1.3 m.

3. Dispositif selon l'une des revendications précédentes, **caractérisé en ce que** le moyen d'activation (4) comprend un bouton poussoir ou un capteur.

4. Dispositif selon l'une des revendications précédentes, **caractérisé en ce que** l'unité de contrôle (9) comprend des moyens de temporisation (13) aptes à désactiver le dispositif si après réception du signal d'activation l'unité de contrôle ne reçoit aucun signal de présence pendant un intervalle de temps prédéterminé.

5. Dispositif selon la revendication 4, **caractérisé en ce que** les moyens de temporisation (13) sont aptes à désactiver le dispositif un temps prédéterminé après l'activation des premiers moyens de pulvérisation (6, 16) et/ou des seconds moyens de pulvérisation (7, 17).

6. Dispositif selon l'une des revendications précédentes, **caractérisé en ce que** les moyens de stockage comprennent deux réservoirs (18).

7. Dispositif selon l'une des revendications précédentes, **caractérisé en ce que** les moyens de stockage (18) comprennent des moyens de mesure (10) raccordée à l'unité de contrôle et permettant de mesurer le remplissage desdits moyens de stockage.

8. Dispositif selon l'une des revendications précédentes **caractérisé en ce que** les premiers moyens de détection (11) comprennent un ou plusieurs capteurs infrarouges, inductifs, capacitifs ou à effet Hall.

9. Dispositif selon l'une des revendications précédentes, **caractérisé en ce que** les seconds moyens de détection comprennent une pédale mobile (8) pouvant se déplacer sous l'effet d'une pression exercée par le pied d'un utilisateur entre une position de repos dans laquelle aucun signal de présence n'est transmis à l'unité de contrôle et une position de travail dans laquelle le deuxième signal de présence est transmis à l'unité de contrôle.

10. Dispositif selon l'une des revendications précédentes **caractérisé en ce que** les seconds moyens de détection (12) comprennent un relais électromécanique.

11. Dispositif selon l'une des revendications précédentes, **caractérisé en ce que** les premiers et les deuxièmes moyens de pulvérisation comprennent chacun un doseur (16, 17) piloté par l'unité de contrôle permettant de varier la pression et/ou le débit de pulvérisation.

12. Dispositif selon l'une des revendications précédentes, **caractérisé en ce que** les premiers et les seconds moyens de pulvérisation (6, 7) comprennent plusieurs buses de pulvérisation.

13. Dispositif selon l'une des revendications précédentes, **caractérisé en ce qu'**il comprend un moyen d'affichage (5) piloté par l'unité de contrôle et apte à afficher une indication du remplissage des moyens de stockage (18).

14. Dispositif selon l'une des revendications précédentes, **caractérisé en ce que** le moyen d'alimentation est apte à être raccordé au secteur ou consiste en une batterie, une pile sèche ou un panneau photovoltaïque.
